# EUROPEAN PATENT APPLICATION

(11) **EP 4 283 632 A1**
(43) Date of publication of application: **29.11.2023**
(21) Application number: 22175720.6
(22) Date of filing: 27.05.2022
(51) Int. Cl.: G21F 7/005, A61B 6/10

(54) **ACCESS AND SAFETY CONTROL FOR RADIATION SHIELDING AREA**

(71) Applicant: Koninklijke Philips N.V., 5656 AG Eindhoven (NL)
(72) Inventor: LEUSSLER, Christoph Günther, Eindhoven (NL); VOGTMEIER, Gereon, Eindhoven (NL); WEISS, Steffen, 5656AG Eindhoven (NL); MYSORE SIDDU, Dinesh, 5656AG Eindhoven (NL)
(74) Representative: Philips Intellectual Property & Standards

(57) **Abstract**

The present invention relates to radiation shielding. In order to provide an access to a radiation shielding area without disturbing the imaging procedure, while still ensuring good enough radiation safety, an access limiting arrangement is proposed for providing an access to a radiation shielding area. The access limiting arrangement comprising a plurality of radiation-shielding devices defining an entrance of the radiation shielding area. The plurality of radiation-shielding devices are configured and arranged in such a way that a person and/or an object is able to access the radiation shielding area during imaging, while passage of a radiation is substantially blocked by at least one of the plurality of radiation-shielding devices such that the radiation is reduced to the acceptable level.

## Description

### FIELD OF THE INVENTION

The present invention relates to radiation shielding, and in particular to an access limiting arrangement and an access control system for providing a controlled access to a radiation shielding area.

### BACKGROUND OF THE INVENTION

Usually, the access to a diagnostic and/or therapy room is not feasible during imaging. For example, standard magnetic resonance (MR) imaging sessions are usually performed under full control of experienced staff members, such as positioning the patient on the MR patient support, applying radiofrequency (RF) coils, selecting, adapting, and running the specific set of scans, performing some immediate image quality control, archiving the images, and dismissing the patient from the MR suite. Because MR sessions are typically relatively long, this represents a large cost factor in radiology. During an MRI scan the access to the MR scanner is not feasible due to RF receiver interference and MR uncontrolled EMC transmitter interfering with hospital equipment.

### SUMMARY OF THE INVENTION

It is thus an object of the present invention to provide an access to a radiation shielding area without disturbing the imaging procedure, while still ensuring radiation safety.
The object of the present invention is solved by the subject-matter of the independent claims. Further embodiments and advantages of the invention are incorporated in the dependent claims. Furthermore, it shall be noted that all embodiments of the present invention concerning a method might be carried out with the order of the steps as described, nevertheless this has not to be the only and essential order of the steps of the method as presented herein. The method disclosed herein can be carried out with another order of the disclosed steps without departing from the respective method embodiment, unless explicitly mentioned to the contrary hereinafter.

According to a first aspect of the present invention, there is provided an access limiting arrangement for providing an access to a radiation shielding area. The access limiting arrangement comprising a plurality of radiation-shielding devices defining an entrance of the radiation shielding area. The plurality of radiation-shielding devices are configured and arranged in such a way that a person and/or an object is able to access the radiation shielding area during imaging, while passage of a radiation is substantially blocked by at least one of the plurality of radiation-shielding devices such that the radiation is reduced to an acceptable level.

The inventors of the present invention have found out that the disadvantages of the actual situation in a imaging suite, such as a diagnostic, therapy, or interventional imaging suite, is the limited option to enter or leave the room without disturbing the imaging procedure while still ensuring good enough radiation safety. The inventors of the present invention have also found out that there may be a need for a controlled access for magnetic resonance-positron emission tomography (MR-PET) and computed tomography-positron emission tomography (CT-PET) during tracer patient preparation.

Towards this end, an access limiting arrangement is proposed for providing an access to a radiation shielding area. For example, the proposed access limiting arrangement may allow controlled and safe access to diagnostic systems, which make use of active radiation for imaging and therapy. Examples of the diagnostic systems may include, but are not limited to, X-ray, computed tomography (CT), MRI, hyperthermia, linear accelerator (LINAC), MR-PET, and CT-PET. For example, the proposed access limiting arrangement may allow controlled and safe access during CT/MR-PET tracer administration, or MRI contrast agents for workflow optimization.

Additionally, access limiting arrangement for a interventional (e.g., MRI, X-ray, etc.) suite may also allow staff to enter and leave during service without disturbing image quality or stop the clinical interventional workflow and provide safety for patient and staff. It will be appreciated that besides radiation shielding, utility services, such as airflow, temperature, disinfection may also be available that still allow the "doorless" entrance.

The access limiting arrangement comprises a plurality of radiation-shielding devices defining an entrance of the radiation shielding area. The plurality of radiation-shielding devices are configured and arranged in such a way that a person and/or an object is able to access the radiation shielding area during imaging, while passage of a radiation generated by the medical imaging apparatus is substantially blocked by at least one of the plurality of radiation-shielding devices, such as no or only limited radiation less than a threshold level can enter or leave the radiation-shielding area. In other words, the radiation should be reduced down to an acceptable defined level according to e.g., local regulations. It should be noted that the term "substantially blocking the passage of radiation" may refer to mitigating or eliminating the influence of direct radiation and indirect radiation. For example, the indirect radiation may comprise scattered radiation with multiple reflections.

Depending on the imaging modality, the radiation may include one or more of an X-ray radiation, Gamma radiation, a radiofrequency (RF) radiation, and an acoustic radiation. Therefore, the radiation-shielding device may comprise different materials depending on the imaging modality. The type and amount of the shielding material to attenuate the radiation is dependent upon the energy of the radiation, the material's composition, and the material's density. For example, to mitigate or eliminate the influence of gamma radiation, lead, copper, stainless steel, and other high-Z materials may be used as shielding materials for the radiation-shielding devices. For example, in order to prevent noise of radio frequency (RF) from entering into the MRI scanner and distorting the image, copper, steel, aluminium, and other RF shielding material may be used for the radiation-shielding devices.

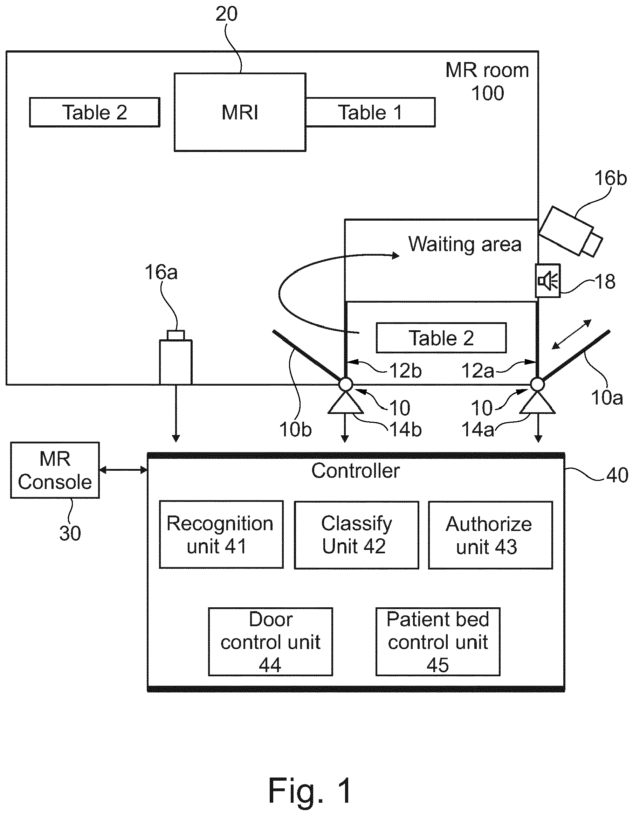

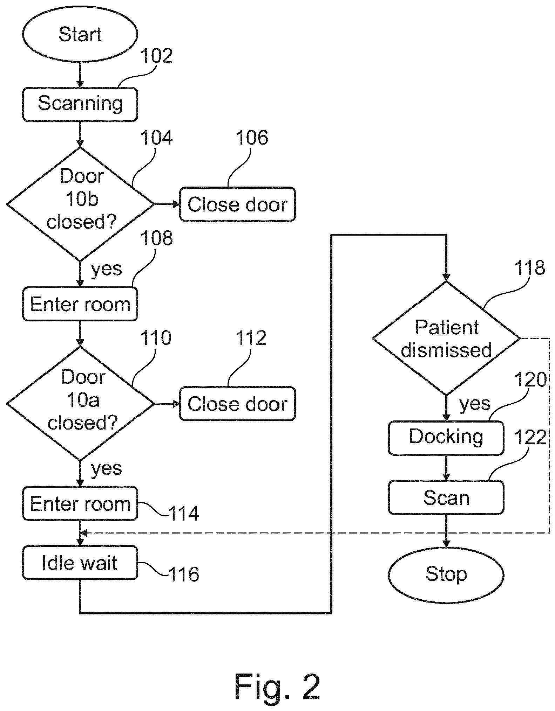

In some examples, the plurality of radiation-shielding devices comprises at least two radiation-shielding doors arranged to open and close the entrance of the radiation shielding area. The at least two radiation-shielding doors are configured to open and close sequentially such that the passage of the radiation generated by the medical imaging apparatus is substantially blocked by at least one of the radiation-shielding doors. The at least two radiation-shielding doors may include any type of doors, which people and/or objects can pass through. Example of the radiation-shielding doors may include, but are not limited to, door entrance with a shutter, sliding doors, revolving doors, and swinging doors. Each radiation-shielding doors may be locked with a respective electronic locking mechanism. A controller may release the electronic locking mechanism, which permits a one or more radiation-shielding doors to be opened by a person or a door operating system. The controller may detect the locking status of the radiation-shielding doors to ensure that at least one of the radiation-shielding doors is in a locking state to provide radiation shielding. This will be explained in detail hereinafter and in particular with respect to the examples shown in Figs. 1 and 2.

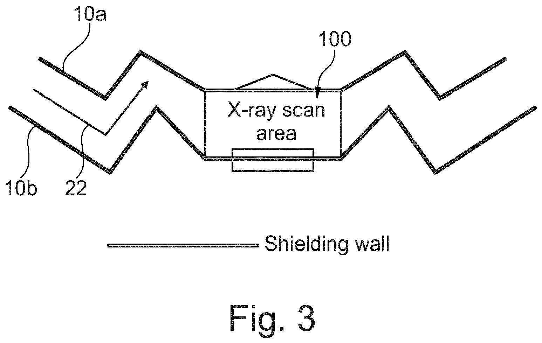

In some examples, the plurality of radiation-shielding devices comprises two radiation-shielding walls that define a path to the radiation shielding area, thereby providing a "walk through" or "doorless" entrance The path may be a curved path such that the passage of the radiation generated by the medical imaging apparatus is substantially blocked by at least one of the two radiation-shielding walls. Alternatively or additionally, one or more radiation-shielding curtain-like structures are provided and arranged along the path. The radiation-shielding walls may provide mobile radiation shielding solutions. For example, the radiation-shielding walls may be transported and used in the nursing homes to provide radiation shielding. This will be explained in detail hereinafter and in particular with respect to the examples shown in Figs. 3 to 5.

The selection of the radiation-shielding doors and the radiation-shielding walls as described herein may be dependent on the imaging modality and corresponding regulations. For example, in CT there is normally and interlock switch at the door that stops X-ray if the door is opened, the plurality of radiation-shielding device may comprise at least radiation-shielding doors that enable more comfortable access to the room e.g., without opening a door and interrupting the procedure but still ensuring radiation protection to the outside. In pure diagnostic rooms, a kind of "walk through" option with the above-described radiation-shielding walls e.g., in DXR could be attractive and still fulfilling radiation shielding requirements. The "walk though" option is also applicable for a CT/MR-PET tracer administration area. In some implementations, the radiation-shielding devices as described herein may be arranged to provide three-dimensional radiation shielding. Thus, the radiation-shielding device may include floor and ceiling as part of the system in case it would be required in dependence upon local regulations and shielding requirements. For example, instead of or in addition to using a curved structure in the horizontal way as shown in Figs. 3 to 5, it is possible to provide an access using a stair up and stair down. So the stair provides also a blocking mass for radiation shield. In this way, no direct radiation could go out of the room and radiation to the outside of the "walls area" would have to undergo "multiple scattering events" which would already minimize the dose.

With the access limiting arrangement as described herein, a person and/or an object may access the imaging theatre even if the system is actively radiating, as the radiation is substantially blocked by the access limiting arrangement such as no or a limited amount of radiation would go out of the imaging theatre. For example, in MRI, the access limiting arrangement may be used to reduce electromagnetic radiation and image degradation due to electronic environmental noise. For example, with the access limiting arrangement as described herein, interventional suite using MRI or X-Ray need to have free access for staff without being constrained during imaging. Additionally, the access limiting arrangement as described herein may allow access for the next patient to an MR room even the MR session is not yet finished or completely finished, thereby improving a workflow and patient throughput. The access limiting arrangement may also allow controlled access for CT/MR-PET during tracer patient preparation and administration.

According to an embodiment of the present invention, the plurality of radiation-shielding devices comprises at least two radiation-shielding doors arranged to open and close the entrance of the radiation shielding area. The at least two radiation-shielding doors are configured to open and close sequentially such that the passage of the radiation generated by the medical imaging apparatus is substantially blocked by at least one of the radiation-shielding doors such that the radiation is reduced to the acceptable level.

In addition, the at least two radiation-shielding doors may be configured to open and close sequentially during active imaging. For example, the at least two radiation shielding doors may be configured to open and close sequentially if an imaging or an inferential procedure is about to start.

The at least two radiation-shielding doors may comprise any type of doors, which people and objects can path through.

In some examples, the radiation-shielding doors may include one or more planar doors that open and close with hinges on the top, bottom or side, and are operated manually or automatically.

In some examples, the radiation-shielding doors may comprise one or more planner doors that slide up, down or sideways and are operated manually or automatically.
In some examples, the radiation-shielding doors may include one or more folding or rolling doors that are operated manually or automatically.

In some examples, the radiation-shielding doors may comprise one or more rotating doors or turnstiles, which partially or fully occupy the opening and are operated manually or automatically.

In some examples, the radiation-shielding doors may combine any combination of the above-described doors.

According to an embodiment of the present invention, the plurality of radiation-shielding devices comprises two radiation-shielding walls that define a path therebetween to the radiation shielding area.

According to an embodiment of the present invention, the path is a curved path such that the passage of the radiation is substantially blocked by at least one of the two radiation-shielding walls such that the radiation is reduced to the acceptable level.

For example, the path may have a certain geometry that the radiation is substantially blocked such that no (or only limited multi scatter) radiation would go out of the room, but the continuous path allows a person and/or an object to enter or leave the radiation-shielding area. This will be explained in detail hereinafter and in particular with respect to the example shown in Fig. 3.

According to an embodiment of the present invention, the curved path comprises a zigzag path and/or a wavy path.

According to an embodiment of the present invention, the two radiation-shielding walls are foldable. In a folding state the curved path is formed by one or more folding sections.

The foldable geometry may be mechanically linked to actuators to allow supported movement and translation of the shielding structure.

According to an embodiment of the present invention, a number of folding sections is dependent on an intensity of the radiation and/or by a required shielding demand.

The number of folding sections may be also dependent on a desired radiation reduction. For example, if the radiation is required to be reduced to a lower level, the curved path may have more folding sections.

For example, more folding sections may allow for higher radiation intensity. In the imaging room, more elements will decrease radiation dose level.

According to an embodiment of the present invention, the plurality of radiation-shielding devices comprises one or more radiation-shielding curtain-like structures arranged along the path.

The one or more radiation-shielding curtain-like structures are flexible sections, which allow access through curtain like structures. In case the elements are shorter, these radiation-shielding curtain-like structures may be configured as fixed blocking sections.

According to an embodiment of the present invention, the radiation shielding area comprises one or more of: a scanner area, where the medical imaging apparatus is located, a tracer administration area, and a contrast agent administration area.

According to an embodiment of the present invention, the radiation comprises one or more of:
- an X-ray radiation;
- a Gamma ray radiation;
- a radiofrequency radiation; and
- an acoustic radiation.

According to a second aspect of the present invention, there is provided an access control system for providing an access to a radiation shielding area. The access control system comprises a controller and an access limiting arrangement according to the first aspect and any associated example. The access limiting arrangement comprises a plurality of radiation-shielding devices. The controller is configured to control the access limiting arrangement to be configured and arranged in such a way that a person and/or an object is allowed to access the radiation shielding area, while passage of a radiation is substantially blocked by at least one of the plurality of radiation-shielding devices such that the radiation is reduced to an acceptable level.

According to an embodiment of the present invention, the access control system further comprises a sensor arrangement comprising one or more sensors to monitor an event and/or a condition in the radiation shielding area. The controller is configured to apply one or more access rules to the monitored event and/or condition to determine whether the radiation shielding area is ready for an entrance of the person and/or the object.

According to an embodiment of the present invention, the monitored event and/or condition comprises one of more of:
- an intensity of the radiation;
- a frequency band of the radiation;
- an access right for the person and/or the object;
- a scan status;
- a status of the plurality of radiation-shielding devices; and
- a status of patient preparation.

According to an embodiment of the present invention, the access control system further comprises at least one indicator. The at least one indicator is configured to indicate a status of preparation of the radiation shielding area for the entrance of the person and/or the object.

According to an embodiment of the present invention, the object comprises an autonomous patient transport device. The controller is configured to generate a signal triggering the autonomous patient transport device to transport a patient to the radiation shielding area.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig 1 illustrates an exemplary access limiting arrangement in form of radiation-shielding doors for providing an access to a radiation shielding area.
Fig. 2 illustrates an exemplary workflow for controlling a door operating system for the access of the self-driving patient bed during imaging.
Fig. 3 illustrates an example of an access limiting arrangement in form of radiation-shielding walls for providing an access to a radiation shielding area.
Fig. 4 illustrates another example of an access limiting arrangement in form of radiation-shielding walls for providing an access to a radiation shielding area.
Fig. 5 illustrates a further example of an access limiting arrangement in form of radiation-shielding walls for providing an access to a radiation shielding area.
Fig. 6 illustrates an example of an access limiting arrangement in form of radiation-shielding walls for providing an access to tracer/contrast administration area.
Fig. 7 illustrates a scheduling of conventional MR room (A) and with smart controlled workflow concept (B).
Fig. 8 shows an example of an access control system for a plurality of imaging modalities.
Fig 9 illustrates an example of a sequence of different imaging scans and therapy sessions.

### DETAILED DESCRIPTION OF EMBODIMENTS

Fig. 1 illustrates an example of an access limiting arrangement 10 for providing an access to a radiation shielding area 100. In this example, the radiation shielding area 100 is an MR room, where an MRI scanner 20 is located. A patient table, such as Table 1 or Table 2 may be docked to the MRI scanner 20 alternatively. The MRI scanner 20 is controlled by an MR console 30. The MR console 30 may be located in a control room (not shown), which is a room adjacent to the MR room 100 with direct visibility of the patient for remote control of the equipment, and review and reporting of procedure images.

Access to the MRI room 100 is through two independent entries 12a, 12b. Each entry may include a controlled door way with an electronic locking mechanism (not shown). The electronic locking mechanism may release when access is granted, which permits the radiation-shielding doors 10a, 10b to be opened by a person or a door operating system, such as actuators of the radiation-shielding doors.

Although the radiation-shielding doors 10a, 10b of the illustrated example are swinging doors, it will be appreciated that the radiation-shielding doors may include any suitable type of radiation-shielding doors. In some examples, the radiation-shielding doors may include one or more planar doors that open and close with hinges on the top, bottom or side, and are operated manually or automatically. In some examples, the radiation-shielding doors may comprise one or more planner doors that slide up, down or sideways and are operated manually or automatically. In some examples, the radiation-shielding doors may include one or more folding or rolling doors that are operated manually or automatically. In some examples, the radiation-shielding doors may comprise one or more rotating doors or turnstiles, which partially or fully occupy the opening and are operated manually or automatically. In some examples, the radiation-shielding doors may comprise a door entrance with a shutter, which may be operated manually or automatically.

In order to allow a person (e.g., patients, healthcare practitioners, persons accompanying patients, and other visitors,) and/or an object (e.g., medical equipment) to enter the MR room 100 without disturbing the imaging procedure (e.g., interlock of door stops the procedure), while still ensuring good enough radiation safety, the two radiation-shielding doors 10a, 10b are configured to open and close sequentially such that the passage of the radiation generated by the MRI scanner 20 is substantially blocked by at least one of the radiation-shielding doors such that the radiation is reduced to the acceptable level. In some examples, the at least two radiation-shielding doors 10a, 10b may be configured to open and close sequentially during active imaging. In some examples, the at least two radiation shielding doors may be configured to open and close sequentially if an imaging or an inferential procedure is about to start. In other words, during active imaging and if an imaging or an inferential procedure is about to start, the two radiation-shielding doors 10a, 10b cannot be opened simultaneously. For example, in response to detecting that the radiation-shielding door 10a is closed, the electronic locking mechanism for the radiation-shielding door 10b may be released, which permits the radiation-shielding door 10b to be opened by a person or a door operating system. Likewise, in response to detecting that the radiation-shielding door 10b is closed, the electronic locking mechanism for the radiation-shielding door 10a may be released, which permits the radiation-shielding door 10a to be opened by a person or a door operating system. In this way, a person and/or an object is able to enter or leave the MR room 100 during imaging, while passage of the RF and acoustic radiation generated by the MR scanner 20 is substantially blocked by at least one of the two radiation-shielding doors 10a, 10b such that the radiation is reduced to the acceptable level. The open or closed status of two radiation-shielding doors 10a, 10b may be detected by two door status sensors 14a, 14b, respectively. And this information may be used by the imaging system e.g., for image processing to take status into account in case of different influences.

The MR room 100 may be monitored by a video camera 16a, which may be placed overhead of the MR room 100, or placed at any convenient location with a clear view of the MR room 100, in particular a clear view of persons and objects in the MR room 100. The camera position of the video camera 16a may be adjusted manually or by motor drive that is locally or remotely controlled. Another video camera, such as video camera 16b shown in Fig. 1, may be arranged to provide a clear view of the entries. The video cameras 16a, 16b may be of an analogue or digital type. The video cameras 16a, 16b may be a 3D camera. For example, the 3D camera may be a range camera used to produce a two-dimensional image showing the distance to points in a scene from a specific point. In another example, the 3D camera is a stereo camera with two or more lenses with separate image sensors or film frame for each lens, which allows the camera to simulate human binocular vision, and therefore capture three-dimensional images. Although Fig. 1 may show a limited number of video cameras by way of example, it can be appreciated that a greater number of video cameras, such as two, three, four, or more video cameras, may be employed for a given implementation to achieve a better view of the MR room 100 and the entries 12a, 12b.

The door status sensors 14a, 14b, the video cameras 16a, 16b, and the MR console 30 are communicatively connected to the controller 30. The communication may be wired or wireless. The controller 40 may comprise various physical and/or logical components for communicating and manipulating information, which may be implemented as hardware components (e.g. computing devices, processors, logic devices), executable computer program instructions (e.g. firmware, software) to be executed by various hardware components, or any combination thereof, as desired for a given set of design parameters or performance constraints. In some implementations, the controller 40 may be embodied as, or in, a device or apparatus, such as a server, workstation, or mobile device. The controller 40 may comprise one or more microprocessors or computer processors, which execute appropriate software. The software may have been downloaded and/or stored in a corresponding memory, e.g. a volatile memory such as RAM or a non-volatile memory such as flash. The software may comprise instructions configuring the one or more processors to perform the functions described herein. It is noted that the controller 40 may be implemented with or without employing a processor, and also may be implemented as a combination of dedicated hardware to perform some functions and a processor (e.g. one or more programmed microprocessors and associated circuitry) to perform other functions. For example, the functional units of the controller 40 may be implemented in the device or apparatus in the form of programmable logic, e.g. as a Field-Programmable Gate Array (FPGA). In general, each functional unit of the apparatus may be implemented in the form of a circuit.

During a running scan, the controller 40 may receive data obtained from the door status sensors 14a, 14b, the video camera 16, and the MR console 30 to monitor an event and/or a condition in the radiation shielding area. For example, the controller 40 may determine a frequency spectrum and intensity of RF radiation based on the scan protocol and the scan status obtained from the MRI scanner 20. The controller 40 may determine the door status of the radiation-shielding doors 10a, 10b based on the data obtained from the door status sensors 14a, 14b. The video camera 16 may be used to detect the position of a person and/or an object in the MR room 100.

The controller 40 may be configured to apply one or more access rules to the monitored event and/or condition to determine whether the radiation shielding area is ready for an entrance of the person and/or the object. In the example of MRI room shown in Fig. 1, the controller 40 may determine the access based on the intensity spectrum and the intensity of the RF radiation generated by the MRI scanner 20 and access rights for the person and/or the object. For example, the intensity of the radiation may be measured by one or more sensors, which may be located between the two radiation-shielding doors 10a, 10b, inside the MR room 100, and outside the MR room. For example, the person may be classified as different user groups, such as trained, untrained, visitor, and unknown and the corresponding access right can be determined based on the user group. Classification can be realized by machine learning algorithm and can be implemented on trained neuronal network. For example, the object may be classified as MR safe, MR conditional, MR unsafe, and unknown. For example, an object recognized as a ventilator can be classified as MR unsafe. Unknown can be defined as a subclass of MR unsafe or a separate class according to site policies.

For example, during a running scan, there may be a clinical need for a staff to enter the MRI room. For example, there may be a need during MR imaging of sedated patients (e.g., children) to provide access during the scan for an anaesthetist. For example, there may be a need for a staff to control and monitor patient during long MRI scans. In this example, the video camera 16b may acquire images of persons approaching the entry 12a and transmit the acquired images to the controller 40. The controller 40 may comprise a recognition unit 41 configured to recognize the imaged person using e.g., facial recognition, pedestrian recognition, skin recognition, clothing recognition, and the like. Recognition may include comparison with person feature stored in a database. The recognized person can be classified according to local MRI safety guidelines. For example, a person recognized by the recognize unit and matched with an identity in the data store is assigned to a class of trained persons, which may be further classified as person authorized to have access to the MR radiation shielding area, but not the MR environment, person authorized to have free access to the MR environment. Recognized by the recognize unit and not matched with an identity can be classified by the classify unit 42 as unknown, visitor, or untrained and corresponding access rule defined for the class. An authorize unit 43 determines access to the MR room 100 based on each classified object and person and the intensity of the RF radiation. When the staff is authorized to enter the MR room during imaging, the staff may be warned to use ear protection and to take care of RF sensitive devices e.g., at the entry 12a. For example, an audible indicator may provide verbal feedback such as verbal statements or instructions. For example, an alarm sound of short duration three times may precede a verbal statement delivered "the unit is operated, please wear ear protection. Persons fitted with heart pacemakers must not enter." A door control unit 44 controls the two radiation-shielding doors 10a, 10b. As described above, the door control unit 44 configures the two radiation-shielding doors 10a, 10b to open and close sequentially such that the passage of the radiation generated by the MRI scanner 20 is substantially blocked by at least one of the radiation-shielding doors 10a, 10b. For example, when the staff is authorized to enter the MRI room 100 during imaging, the door control unit 44 firstly detects whether the radiation-shielding door 10b is closed via the door status sensor 14b. If the radiation-shielding door 10b is open, the door control unit 44 may control a door operating system (e.g., actuators of the doors) to close the radiation-shielding door 10b. In response to detecting that the radiation-shielding door 10b is closed, the door control unit 44 releases the electronic locking mechanism for the radiation-shielding door 10a, which permits the radiation-shielding door 10a to be opened by the staff or a door operating system. When the staff is approaching the entry 12b, the radiation-shielding door 10a may be closed by the staff or the door operating system. In response to detecting that the radiation-shielding door 10a is closed via the door status sensor 14a, the door control unit 44 then releases the electronic locking mechanism for the radiation-shielding door 10b, which permits the radiation-shielding door 10b to be opened by the staff or the door operating system. In this way, the staff is able to enter the MR room 100 during imaging, while passage of the RF and acoustic radiation is substantially blocked by at least one of the two radiation-shielding doors 10a, 10b.

In another example, during imaging, there may be a need for a person (e.g., a staff, relatives accompanying the patient) to leave the MR room without stopping the scan. In this example, the video camera 16a may monitor the position of the person in the MR room 100, and transmit the information to the controller 40. If it is determined that the person is approaching the entry 12b, the door control unit 44 may detect whether the radiation-shielding door 10a is closed via the door status sensor 14a. If the radiation-shielding door 10a is open, the door control unit 44 may control the door operating system (e.g., actuators of the doors) to close the radiation-shielding door 10a. In response to detecting that the radiation-shielding door 10a is closed, the door control unit releases the electronic locking mechanism for the radiation-shielding door 10b, which permits the radiation-shielding door 10b to be opened by the person or the door operating system. When the person enters the entry 12b, the radiation-shielding door 10b may be closed by the person or the door operating system. In response to detecting that the radiation-shielding door 10b is closed via the door status sensor 14b, the door control unit 44 may release the electronic locking mechanism for the radiation-shielding door 10a, which permits the radiation-shielding door 10a to be opened by the person or the door operating system. In this way, the person is able to leave the MR room 100 during imaging, while passage of the RF and acoustic radiation is substantially blocked by at least one of the two radiation-shielding doors 10a, 10b.

In a further example, during imaging, there may be a need for an object to enter the scanner room. For example, some patient beds may have sensors and transmitters integrated so that RF doors and access may be performed automatically, which may be beneficial for autonomous imaging. For example, as shown in Fig. 1, a self-driving patient bed, also referred to as Table 2, may be used transfer a second patient to the MR room 100. The self-driving patient bed is normally registered and allowed to enter the MR room.

Fig. 2 illustrates an exemplary workflow for controlling a door operating system, e.g., actuators of the radiation-shielding doors for the access of the self-driving patient bed during imaging. In step 102, a self-driving patient bed, i.e., Table 1, is docked to the MRI scanner, which is scanning a first patient on Table 1. The controller 40 may obtain information about the MR scan status from the MR console. If the MR scan status indicates that a next patient can enter the MR room, a patient bed control unit 45 of the controller 40 may generate a signal to trigger the self-driving patient bed, i.e., Table 2, to transfer the second patient to the entry 12a. The recognition unit 41 may receive images of objects approaching the entry 12a acquired by the video camera 16b, and recognize the object using an objection recognition approach including, but not limited to, shape recognition, texture recognition, edge detection, label recognition, and the like. The classify unit 42 of the controller 40 may classify the object as MR safe, MR unsafe, and unknown. If the self-driving patient bed, i.e., Table 2, arrives at the entry 12a and classified as MR safe, the authorize unit 43 authorizes the self-driving patient bed, i.e., Table 2, to enter the MR room.

In step 104, the door control unit 44 receives data from the door status sensor 14b and determines whether the radiation-shielding door 10b is closed. If the radiation-shielding door 10b is in an open state, the door control unit 44 may operate the door operating system (e.g., actuators of the doors) to close the radiation-shielding door 10b in step 106.

In step 108, in response to detecting that the radiation-shielding door 10b is in a closed state, the door control unit 44 may release the electronic locking mechanism for the radiation-shielding door 10a, which permits the radiation-shielding door 10a to be opened by the door operating system. And the self-driving patient bed, i.e., Table 2, can transfer the next patient into the entrance area between two radiation-shielding doors 10a, 10b.

In step 110, the door control unit 44 receives data from the door status sensor 14a and determines whether the radiation-shielding door 10a is closed. If the radiation-shielding door 10a is in an open state, the door control unit 45 may operate the door operating system (e.g., actuators of the doors) to close the radiation-shielding door 10a in step 112.

In step 114, in response to detecting that the radiation-shielding door 10a is in a closed state, the door control unit 44 may release the electronic locking mechanism for the radiation-shielding door 10b, which permits the radiation-shielding door 10b to be opened by the door operating system. And the self-driving patient bed can transfer the next patient into the MR room 100. As the first patient on Table 1 is not dismissed, the self-driving patient bed may be parked in a waiting area as shown in Fig. 1 in step 116.

Once the first patient on Table 1 is dismissed in step 118, Table 2 with the second patient can be directly docked to the MRI scanner 20 in step 120, so that the scan can start in step 122. During scan of the second patient the first patient may be transferred back to the patient preparation area. In this example, the patient table, such as Table 1 and Table 2 shown in Fig. 1, may be docked at both sides to provide faster access. The magnet may be equipped with docking and control monitor equipment at both sides.

Although the plurality of radiation-shielding doors are described with respect to MRI room, it will be appreciated that the radiation-shielding doors are also applicable for other medical radiation-shielding rooms, such as radiology, diagnostic imaging, nuclear medicine, cath labs, OR, and special procedure rooms. Depending on the imaging modality, the plurality of radiation-shielding doors may be configured to block or suppress one or more of the following radiations: X-ray, Gamma, and RF radiations.

Fig. 3 illustrates an example of an access limiting arrangement 10 for providing an access to a radiation shielding area 100. The radiation shielding area 100 of the illustrated example is an X-ray scan area, where an X-ray imaging device (not shown) is located.

The access limiting arrangement 10 of the illustrated example comprises two radiation-shielding walls 10a, 10b that define a path 22 therebetween, indicated with an arrow, to the X-ray scan area 100, thereby providing a "walk through" or "doorless" entrance.The path 22 may be a curved path such that the passage of the radiation generated by the X-ray device is substantially blocked by at least one of the two radiation-shielding walls 10a, 10b. For example, special path like an optical trap may be arranged for a person and/or an object to enter or leave the X-ray scan area 100 without a door. The path 22 may have a certain geometry that X-ray scatter is substantially blocked such that no (or only limited multi scatter) radiation would go out of the room, but the continuous path 22 allows a person and/or an object to enter or leave the X-ray scan area 100. In other words, no direct radiation could go out of the room, and radiation to the outside of the "walls area" would have to undergo "multiple scattering events" which would already minimize the dose. For example, the path 22 may have a zigzag form or wavy form.

In some examples, the radiation-shielding walls 10a, 10b may be foldable in length, so that the geometry can be changed. During imaging, the radiation-shielding walls 10a, 10b may be folded as shown in Fig. 3. In a folding state the curved path is formed by one or more folding sections. The geometry can be adapted to the X-ray intensity. The number of folding sections is dependent on an intensity of the radiation generated by the medical imaging apparatus and/or by a required shielding demand. More folding sections allow for higher radiation intensity.

In some examples, the foldable structure may be combined with a door, such as sliding or revolving doors. The foldable geometry may be mechanically linked to actuators to allow supported movement and translation of the shielding structure.

In some examples (not shown), instead of using a curved structure in the horizontal way, the radiation-shielding walls may be configured to provide an access using a stair up and stair down. So the stair provides also a blocking mass for radiation shield.

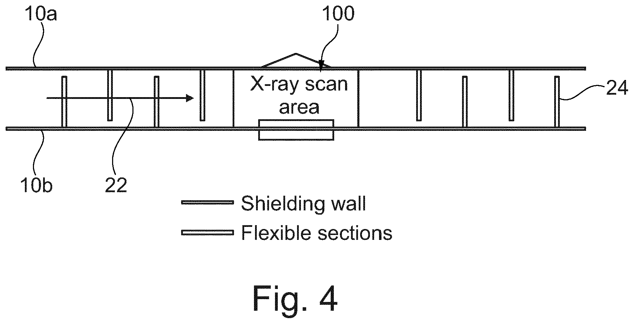

Fig. 4 illustrates another example of an access limiting arrangement 10 for providing an access to a radiation shielding area 100. The radiation shielding area 100 of the illustrated example is an X-ray scan area, where an X-ray imaging device (not shown) is located.

The access limiting arrangement 10 of the illustrated example also comprises two radiation-shielding walls 10a, 10b that define a path 22 therebetween, indicated with an arrow, to the X-ray scan area 100. Unlike the example shown in Fig. 3, the path 22 is a straight path with additional flexible sections 24 along the path 22. Each flexible sections 24 is a radiation-shielding element. These flexible sections 24 may be curtain-like structures, such as lead curtains, which allow a person and/or an object to enter or leave the X-ray scan area, while blocking the passage of the X-ray radiation. For example, the geometry with "shielding flexible wall" may be close to side wall to allow e.g., patients (e.g. also with a wheelchair) enter the diagnostic room.

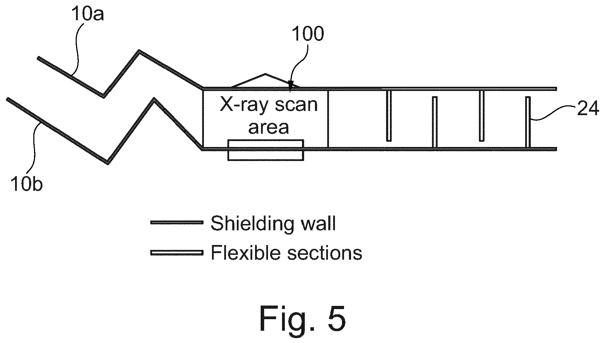

Fig. 5 illustrates a further example of an access limiting arrangement 10 for providing an access to a radiation shielding area 100. The radiation shielding area 100 of the illustrated example is an X-ray scan area, where an X-ray imaging device (not shown) is located. The access limiting arrangement 10 of the illustrated example also comprises two radiation-shielding walls 10a, 10b that define a path therebetween to the X-ray scan area 100.

In this example, the left part of the radiation-shielding walls 10a, 10b are configured to be foldable in length, so that the geometry can be changed. During imaging, the left part of the radiation-shielding walls 10a, 10b may be folded as shown in Fig. 5. The right part of the radiation-shielding walls 10a, 10b define a straight path with additional flexible sections 24 along the path. These flexible sections 24 may be curtain-like structures which allow a person and/or an object to enter or leave the X-ray scan area, but block the passage of the X-ray radiation.

Although the radiation-shielding walls shown in Figs. 3 to 5 are discussed with respect to X-ray scan area, the skilled person will appreciated that the radiation-shielding walls may also be applicable to other diagnostic, therapy, or interventional imaging suite.

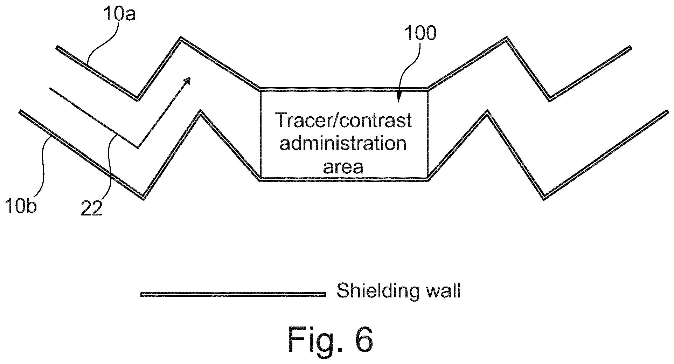

The radiation-shielding walls shown in Figs. 3 to 5 may be transportable and used as mobile radiation shielding solutions. The mobile settings of such a shielding may help in environments, where a fixed shielding infrastructure is not available. For example, in case X-ray imaging goes to the patient (e.g., nursing homes), the radiation-shielding walls may be transported and used in the nursing homes to provide radiation shielding. In some examples, the radiation-shielding walls shown in Figs. 3 to 5 may be used to define other types of radiation shielding area, where no medical imaging apparatus is located. For example, as shown in Fig. 6, the radiation-shielding walls may be used to define a tracer administration area and patient waiting area 100. In tracer administration area, fluorodeoxyglucose (FDG) tracer is injected into a vein or through intravenous (IV) line to distribute the tracer within the human body. In the patient waiting area, patient will wait after the administration of tracer till tracer reaches the body parts that are of interest for PET CT or PET MR scanning, usually it takes 30 to 90 minutes based on the physiological condition of the patients. During the patient waiting time nurse/technician's has to move in and out of the waiting area to monitor the patient. Special wall shielding will help hospital nurse and technicians movements and it also removes the claustrophobic environment (locked inside the lead wall room) to patients.

Although the exemplary radiation-shielding devices shown in Figs. 1 to 5 are described to provide two-dimensional radiation shielding, it will be appreciated that in some implementations, the radiation-shielding devices as described herein may be arranged to provide three-dimensional radiation shielding. Thus, the radiation-shielding device may include floor and ceiling as part of the system in case it would be required in dependence upon local regulations and shielding requirements.

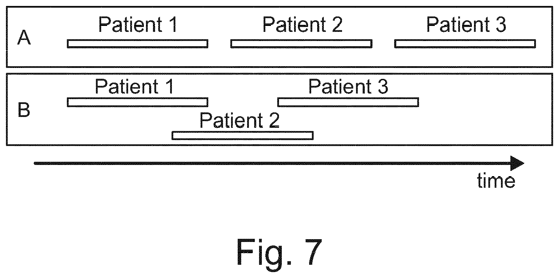

With the above-described access limiting arrangement, it is possible to improve the workflow if the next patient is already prepared in a patient preparation room. Fig. 7 illustrates a scheduling of conventional MR room (A) and with smart controlled workflow concept (B).

In the conventional scanner the patients are transferred to the scanner and in a serial manner (A). This is because that although the next patient is already prepared in a patient preparation room, the access to the MR room is only allowed after the previous patient is dismissed. Therefore, between the previous patient and the next patient there is a time gap to open and close the door.

With the access limiting arrangement described herein, the prepared patient can already enter the diagnostic imaging suite during a scan of the preceding patient (B), e.g., in the manner described in Fig. 2, without disturbing the scan process. In this way, a considerable timesaving and reduced workflow is achieved.

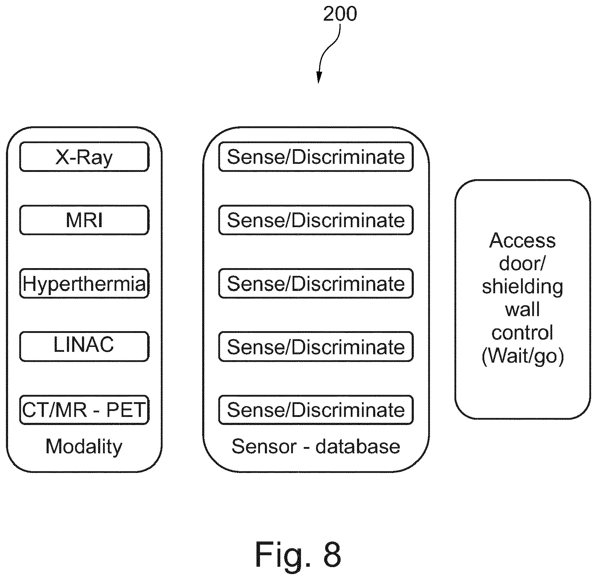

Fig. 8 shows an example of an access control system 200 for a plurality of imaging modalities, such as X-ray, MRI, Hyperthermia, LINAC, and CT/MR-PET. These different diagnostic modalities may be located in an imaging room or in an interventional clinical environment. Each modality may be equipped with a sensor and digitally linked with a controller. Discriminator level may depend on different parameters, such as allowed radiation dose, electromagnetic radiation intensity, distance, urgency, radiation protection of staff, real time imaging during intervention, acoustic noise level of scan, fluoroscopy, etc. For example, access to an X-ray room may be determined based upon the radiation dose and access rights for medical staff or patient.

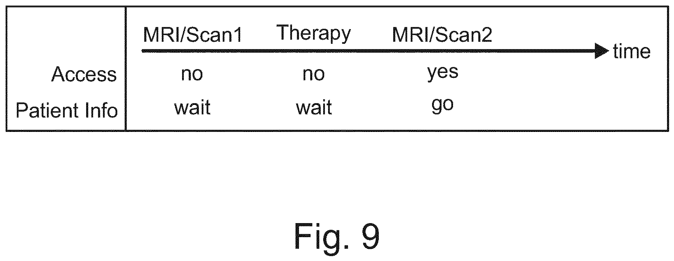

Fig. 9 illustrates an example of a sequence of different imaging scans and therapy sessions. Access may be allowed to patient or staff during defined time interval. As an example, for an interventional suite, where during defined critical time periods access is reduced or forbidden. Scan will automatically resumed if a person leaves the imaging room. Additional inform may be provided to a patient concerning how long to wait to enter the room.

All definitions, as defined and used herein, should be understood to control over dictionary definitions, definitions in documents incorporated by reference, and/or ordinary meanings of the defined terms.
The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An access limiting arrangement (10) for providing an access to a radiation shielding area (100), the access limiting arrangement comprising:
- a plurality of radiation-shielding devices (10a, 10b) defining an entrance of the radiation shielding area, wherein the plurality of radiation-shielding devices are configured and arranged in such a way that a person and/or an object is able to access the radiation shielding area, while passage of a radiation is substantially blocked by at least one of the plurality of radiation-shielding devices such that the radiation is reduced to an acceptable level.

2. The access limiting arrangement according to claim 1,
wherein the plurality of radiation-shielding devices comprises at least two radiation-shielding doors arranged to open and close the entrance of the radiation shielding area; and
wherein the at least two radiation-shielding doors are configured to open and close sequentially such that the passage of the radiation is substantially blocked by at least one of the radiation-shielding doors such that the radiation is reduced to the acceptable level.

3. The access limiting arrangement according to claim 1 or 2,
wherein the plurality of radiation-shielding devices comprises two radiation-shielding walls that define a path (22) therebetween to the radiation shielding area.

4. The access liming arrangement according to claim 3,
wherein the path is a curved path such that the passage of the radiation is substantially blocked by at least one of the two radiation-shielding walls such that the radiation is reduced to the acceptable level.

5. The access limiting arrangement according to claim 4,
wherein the curved path comprises a zigzag path and/or a wavy path.

6. The access limiting arrangement according to claim 4 or 5,
wherein the two radiation-shielding walls are foldable;
wherein in a folding state the curved path is formed by one or more folding sections.

7. The access limiting arrangement according to claim 6,
wherein an amount of folding sections is dependent on an intensity of the radiation and/or by a required shielding demand.

8. The access limiting arrangement according to any one of claims 3 to 7,
wherein the plurality of radiation-shielding devices comprises one or more radiation-shielding curtain-like structures arranged along the path.

9. The access limiting arrangement according to any one of the preceding claims,
wherein the radiation shielding area comprises one or more of:
- a scanner area, where a medical imaging apparatus is located;
- a tracer administration area; and
- a contrast agent administration area.

10. The access limiting arrangement according to any one of the preceding claims,
wherein the radiation comprises one or more of:
- an X-ray radiation;
- a Gamma ray radiation;
- a radiofrequency radiation; and
- an acoustic radiation.

11. An access control system (200) for providing an access to a radiation shielding area, comprising:
- a controller (40); and
- an access limiting arrangement (10) according to any one of the preceding claims;
wherein the access limiting arrangement comprises a plurality of radiation-shielding devices; and
wherein the controller is configured to control the access limiting arrangement to be configured and arranged in such a way that a person and/or an object is allowed to access the radiation shielding area, while passage of a radiation is substantially blocked by at least one of the plurality of radiation-shielding devices such that the radiation is reduced to an acceptable level.

12. The access control system according to claim 11, further comprising:
- a sensor arrangement (14, 16) comprising one or more sensors to monitor an event and/or a condition in the radiation shielding area; and
wherein the controller is configured to apply one or more access rules to the monitored event and/or condition to determine whether the radiation shielding area is ready for an entrance of the person and/or the object.

13. The access control system according to claim 12,
wherein the monitored event and/or condition comprises one of more of:
- an intensity of the radiation;
- a frequency band of the radiation;
- an access right for the person and/or the object;
- a scan status of a medical imaging apparatus;
- a status of the plurality of radiation-shielding devices; and
- a status of patient preparation.

14. The access control system according to claim 12 or 13, further comprising:
- at least one indicator (18);
wherein the at least one indicator is configured to indicate a status of preparation of the radiation shielding area for the entrance of the person and/or the object.

15. The access control system according to any one of claims 12 to 14,
wherein the object comprises an autonomous patient transport device (Table 2); and
wherein the controller is configured to generate a signal triggering the autonomous patient transport device to transport a patient to the radiation shielding area.
